# EUROPÄISCHE PATENTSCHRIFT

(11) **EP 3 322 453 B1**
(45) Veröffentlichungstag und Bekanntmachung des Hinweises auf die Patenterteilung: **10.04.2019**
(21) Anmeldenummer: 16723758.5
(22) Anmeldetag: 19.05.2016
(51) Int. Cl.: A61L 2/20

(54) **VORRICHTUNG ZUM STERILISIEREN VON VERSCHLÜSSEN FÜR BEHÄLTER**
DEVICE FOR STERILIZING CLOSURES FOR CONTAINERS
DISPOSITIF DESTINÉ À STÉRILISER DES ÉLÉMENTS DE FERMETURE POUR DES RÉCIPIENTS

(30) Priorität: 15.07.2015 DE 102015111446
(43) Veröffentlichungstag der Anmeldung: 23.05.2018
(73) Patentinhaber: KHS GmbH, 44143 Dortmund (DE)
(72) Erfinder: DANELSKI, Alexander, 55543 Bad Kreuznach (DE); NIEHR, Thomas, 55583 Bad Münster am Stein Ebernburg (DE)
(86) Internationale Anmeldenummer: PCT/EP2016/061349
(87) Internationale Veröffentlichungsnummer: WO 2017/008941

(56) Entgegenhaltungen:
- EP-A1- 2 511 070
- EP-A1- 2 522 485
- EP-A1- 2 848 384
- WO-A1-2012/000573

## Beschreibung

Die Erfindung bezieht sich auf eine Vorrichtung zum Entkeimen bzw. Sterilisieren von Verschlüssen gemäß dem Oberbegriff des Patentanspruches 1.

Beispielsweise bei Anlagen der Getränkeindustrie ist es üblich und bekannt, die zum Verschließen von gefüllten Flaschen oder anderen Behältern verwendeten Verschlüsse, insbesondere auch kappenartige Verschlüsse, wie Schraubverschlüsse, Flachkappen, Kronkorken usw. vor ihrer Verwendung, d.h. vor dem Aufbringen auf den jeweiligen Behälter zu sterilisieren bzw. zu entkeimen, und zwar u.a. unter Verwendung von Sterilisiermitteln, die aus H2O2-Dampf (verdampfte wässrige Wasserstoffperoxid-Lösung mit ausreichend hoher H2O2-Konzentation) oder aus einem mit H2O2-Dampf angereicherten gas- und/oder dampfförmigen Trägermedium bestehen. Um eine wirksame Sterilisation mit hoher Entkeimungsrate zu erreichen, werden die Verschlüsse vor der Beaufschlagung mit dem Sterilisiermittel vorzugsweise vorgewärmt, beispielsweise auf eine Temperatur im Bereich zwischen 50°C und 85°C und dann nach der Behandlung bzw. Beaufschlagung mit dem gas- und/oder dampfförmigen Sterilisiermittel unter Verwendung eines vorzugsweise erwärmten sterilen gas- und/oder dampfförmigen Mediums, beispielsweise unter Verwendung von vorzugsweise erwärmter steriler Luft getrocknet, sodass die sterilisierten Verschlüsse dann anschließend zum Verschließen von Behältern verwendet bzw. hierfür einer Verschließmaschine zugeführt werden können.

Eine bekannte Vorrichtung zum Sterilisieren von Verschlüssen für Behälter mit H2O2-Aerosol oder Dampf weist zwei um eine gemeinsame vertikale Maschinenachse umlaufend angetriebene Rotoren auf (DE 10 2010 025 541 A1). Jeder Rotor ist in einem Behandlungsraum aufgenommen. An jedem Rotor ist um die Maschinenachse versetzt eine Vielzahl von Verschlussaufnahmen vorgesehen, die jeweils zur Aufnahme einer Vielzahl von Verschlüssen in Reihe ausgebildet sind und sich in einer Achsrichtung parallel zur Maschinenachse erstrecken. Mit den Verschlussaufnahmen werden die Verschlüsse bei umlaufenden Rotoren durch die Behandlungsräume zwischen einer Verschlussaufgabe und einer Verschlussabgabe bewegt. In den Behandlungsräumen erfolgt jeweils eine Beaufschlagung der Verschlüsse mit Heißluft, und zwar im Behandlungsraum des oberen Rotors für ein Vorerwärmen der Verschlüsse und in dem Behandlungsraum des unteren Rotors für ein Aktivieren des Sterilisationsmediums (H2O2-Aerosol) und für ein Trocknen der Verschlüsse. Die Beaufschlagung der Verschlüsse mit dem Sterilisationsmedium erfolgt auf einer parallel zur Maschinenachse orientierten Behandlungsstrecke, die die Verschlussabgabe des oberen Rotors mit der Verschlussaufgabe des unteren Rotors verbindet und durch die die Verschlüsse durch Schwerkraft hindurchbewegt werden.

In der DE 10 2010 052 207 A1 wurde weiterhin eine Vorrichtung vorgeschlagen, die zum Sterilisieren von Verschlüssen durch Beaufschlagung mit UV-Strahlung dient und bei der die Verschlüsse in vertikal ausgerichteten, käfigartigen Verschlussaufnahmen aufgenommen sind, die am Umfang eines um eine vertikale Maschinenachse umlaufend antreibbaren Rotors gebildet und mit ihrer Längserstreckung parallel zur Rotorachse orientiert sind. Die Verschlüsse werden dabei mit dem Rotor bzw. mit den Verschlussaufnahmen auf einer Behandlungsstrecke zwischen einer Verschlussaufgabe und einer Verschlussabgabe einer Vielzahl von mit dem Rotor nicht umlaufenden UV-Strahlern vorbei bewegt.

Aus der DE 10 2012 017 986 A1 ist zudem eine Vorrichtung und Verfahren zum Sterilisieren von Verschlüssen zum Verschließen von Flaschen oder dergleichen Behältern bekannt geworden, insbesondere zum Sterilisieren von kappenartigen Verschlüssen, mit einem Transportsystem zum Bewegen der Verschlüsse durch wenigstens eine Behandlungszone, in der die Verschlüsse sterilisiert werden, wobei das Transportsystem in der wenigstens einen Behandlungszone wenigstens einen um eine Maschinen oder Rotorachse umlaufend antreibbaren Rotor mit einer Vielzahl von am Umfang dieses Rotors ausgebildeten Verschlussaufnahmen aufweist, mit denen die Verschlüsse durch die wenigstens eine Behandlungszone auf einer Behandlungsstrecke zwischen einer Verschlussaufgabe und einer Verschlussabgabe bewegt werden. Dabei sind an der wenigstens einen Behandlungsstrecke eine oder mehrere Applikationsstationen vorgesehen, mit einer auf zumindest eine Verschlussaufnahme gerichteten Sterilisiermittelsprühvorrichtung zum gesteuerten und/oder gezielten Beaufschlagen der einzelnen in der Verschlussaufnahme aufgenommenen und gehaltenen Verschlüsse mit Wasserstoffperoxid- Aerosol/-Dampf, einem Wasserstoffperoxid-Aerosol enthaltenden Sterilisiermittel und/oder einem Gas zum Aktivieren des Wasserstoffperoxids und/oder Trocknen der Oberflächen. Die Verschlüsse werden dabei ruhend in einer Verschlussaufnahme während der gesamten Behandlungszeit gehalten, d. h. dass sich die Verschlüsse in den Verschlussaufnahmen also während der gesamten Behandlungszeit weder relativ zueinander noch zur Verschlussaufnahme bewegen. Mitunter kann es jedoch beispielweise durch das Beaufschlagen der Verschlüsse mit Sterilisiermittel zu einem unerwünschten Verkanten und/oder Verkleben des jeweiligen Verschlusses in der Verschlussaufnahme und/oder benachbarter Verschlüsse kommen.

Weiterhin ist aus der WO 2012/000573 A1 eine Vorrichtung zum Sterilisieren von Verschlüssen für Flaschen mit einem Transportsystem bekannt, bei welchem eine Vielzahl von Verschlüssen in vertikalen Verschlussaufnahmen aufgenommen und durch wenigstens drei Behandlungszonen gefördert werden, wobei in zwei Zonen, jeweils ein umlaufend antreibbarer Rotor mit einer Vielzahl von Verschlussaufnahmen in Form von parallel zur Maschinenachse verlaufenden Verschlussführungsschienen vorgesehen ist. Diese beiden um eine selbe, vertikale Maschinenachse rotierenden Behandlungszonen sind durch eine sich vertikal erstreckenden Verschlussführung, in einer Art Fallrinne, miteinander verbunden. Aufgabe der Erfindung ist es, eine Vorrichtung zum Sterilisieren und/oder Entkeimen von Verschlüssen zum Verschließen von Flaschen oder dergleichen Behältern, insbesondere zum Sterilisieren von kappenartigen Verschlüssen aufzuzeigen, die die Nachteile aus dem Stand der Technik beseitigt, insbesondere zum gesteuerten Lösen von in der Verschlussaufnahme verkanteten und/oder verklebten Verschlüssen ausgebildet ist. Zur Lösung dieser Aufgabe ist eine Vorrichtung entsprechend den Merkmalen des Patentanspruches 1 ausgebildet.

Der wesentliche Aspekt der erfindungsgemäßen Vorrichtung zum Sterilisieren von Verschlüssen zum Verschließen von Flaschen oder Behältern, insbesondere zum Sterilisieren von kappenartigen Verschlüssen, ist darin zu sehen, dass wenigstens einer vertikal ausgerichteten Verschlussaufnahme eine in das Gehäuse zumindest abschnittsweise eingreifende Stellvorrichtung zum Einleiten einer Stellbewegung mittels mindestens eines Stellglieds auf wenigstens einen in dieser Verschlussaufnahme aufgenommenen Verschluss zugeordnet ist, und wobei das mindestens eine Stellglied über wenigstens einen Faltenbalg mit zumindest einer Öffnung des Gehäuses beweglich verbunden ist.

Der besondere Vorteil besteht unter anderem darin, dass mittels der Stellvorrichtung in einer Verschlussaufnahme verkantete und/oder verklebte Verschlüsse gesteuert wieder gelöst werden können, indem auf diesen mittels eines Stellglieds eine Stellbewegung eingeleitet werden kann.

Nachfolgend soll unter dem Begriff "Verschluss" Verschlüsse unterschiedlichster Art verstanden werden, insbesondere kappenartige Verschlüsse, wie Schraubverschlüsse, Flachkappen, Kronkorken usw.

Besonders vorteilhaft ist die Stellvorrichtung in ihrer Längserstreckung im Wesentlichen parallel zur Maschinenachse orientiert ausgebildet und radial zum Rotor beabstandet angeordnet. Dabei kann die Stellvorrichtung auch oberhalb der Verschlussabgabe derart angeordnet sein, dass mittels des mindestens eines Stellgliedes wenigstens ein in der Verschlussaufnahme aufgenommener Verschluss mit einer Stellbewegung beaufschlagbar ist.

Bevorzugt weist das Stellglied zur Einleitung einer Stellbewegung wenigstens einen Stellgliedstoßabschnitt auf, der mit einer Verschlussachse einen spitzen Winkel einschließt, der vorzugsweise zwischen 25°und 85° Grad liegt.

Gemäß einer bevorzugten Ausführungsvariante kann das Stellglied zumindest einen ersten und zweiten Stellgliedträgerabschnitt aufweisen, wobei der zweite Stellgliedträgerabschnitt mit dem Stellgliedstoßabschnitt verbunden ist.

Nach einer weiteren vorteilhaften Ausführungsvariante können an dem zweiten Stellgliedträgerabschnitt mehrere Stellgliedstoßabschnitte angeordnet sein. Es kann vorteilhaft auch vorgesehen sein, dass die Stellgliedträgerabschnitte stabförmig ausgebildet sind.

Gemäß einer weiteren Ausführungsvariante kann vorgesehen sein, dass der zweite Stellgliedträgerabschnitt sich an den ersten Stellgliedträgerabschnitt anschließt, wobei der erste Stellgliedträgerabschnitt und der zweite Stellgliedträgerabschnitt winkelig zueinander angeordnet sind.

Vorteilhafterweise kann das Stellglied mit seinem ersten Stellgliedträgerabschnitt zumindest teilweise in dem Faltenbalg aufgenommen und durch die Öffnung des Gehäuses beweglich geführt sein, vorzugsweise gas- und/oder flüssigkeitsdicht.

Es kann dabei bevorzugt vorgesehen sein, dass der erste Stellgliedträgerabschnitt an einer ersten Seite durch die Öffnung des Gehäuses beweglich zu dieser hindurchgeführt ist, und mit seiner zweiten Seite durch einen Führungsabschnitt des Faltenbalges hindurchgeführt und in dem Führungsabschnitt mechanisch fixiert aufgenommen ist.

Weiterhin kann vorteilhaft vorgesehen sein, dass das Stellglied mit seinem wenigstens einen Stellgliedstoßabschnitt zumindest teilweise in wenigstens einem Faltenbalg aufgenommen ist und durch die wenigstens eine Öffnung des Gehäuses beweglich geführt ist, vorzugsweise gas- und/oder flüssigkeitsdicht. Vorzugsweise kann hierfür jeweils einem Stellgliedstoßabschnitt ein separater Faltenbalg zugeordnet sein, wobei die einzelnen Stellgliedstoßabschnitte über jeweils eine separate Öffnung des Gehäuses geführt und über den jeweiligen Faltenbalg beweglich verbunden sind.

Der Ausdruck "im Wesentlichen" bedeutet im Sinne der Erfindung Abweichungen von jeweils exakten Wert um +/- 10%, bevorzugt um +/- 5% und/oder Abweichungen in Form von für die Funktion unbedeutenden Änderungen.

Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an einem Ausführungsbeispiel näher erläutert. Es zeigen:
- Fig. 1: in seitlich schematischer Darstellung eine Ausführungsvariante der erfindungsgemäßen Vorrichtung zum Sterilisieren von Verschlüssen;
- Fig. 2: eine schematische Draufsicht der Ausführungsvariante der erfindungsgemäßen Vorrichtung zum Sterilisieren von Verschlüssen gemäß Figur 1;
- Fig. 3: eine schematische seitliche Schnittdarstellung eines Ausschnittes der erfindungsgemäßen Vorrichtung zum Sterilisieren von Verschlüssen gemäß Figur 1;
- Fig. 4: in seitlich schematischer Darstellung eine weitere Ausführungsvariante der erfindungsgemäßen Vorrichtung zum Sterilisieren von Verschlüssen;
- Fig. 5: in seitlich schematischer Darstellung eine nochmals weitere Ausführungsvariante der erfindungsgemäßen Vorrichtung zum Sterilisieren von Verschlüssen.

Die in den Figuren allgemein mit 1 bezeichnete Vorrichtung dient zum Sterilisieren und/oder Entkeimen von Verschlüssen 2, beispielsweise von Verschlüssen in Form von Kappen, Schraubkappen, Sportkappen, Flachkappen oder Kronenkorken usw., die zum Verschließen von nicht dargestellten Behältern, z.B. in Form von Flaschen verwendet werden. Die Vorrichtung 1 bildet hierfür bevorzugt ein einer Verschließmaschine zum Verschließen der Behälter vorgeschaltetes Aggregat, aus dem die sterilisierten Verschlüsse 2 unter sterilen bzw. keimfreien Bedingungen der Verschließmaschine zugeführt werden.

Die Vorrichtung umfasst unter anderem ein Gehäuse 3, welches bei der dargestellten Ausführungsform in Draufsicht eine vertikale Maschinen- oder Rotorachse MA umschließende polygonale oder zylindrische Formgebung aufweist, d. h. beispielsweise sechseckförmig ausgebildet sein kann. Der Innenraum 4 des Gehäuses 3 ist dabei mit Ausnahme von Ein - und Auslässen für die Verschlüsse 2, die insbesondere durch eine Verschlussaufgabe 31 bzw. eine Verschlussabgabe 33 gebildet werden können, gegenüber der Umgebung dicht, insbesondere gas- und/oder flüssigkeitsdicht, verschlossen und zwar durch eine die Maschinenachse MA umschließende Umfangswand 5, durch eine obere Gehäusewand 6 sowie eine untere Gehäusewand 7. In der Umfangswand 5 können Inspektionsfenster vorgesehen sein, wobei ebenso eine Ausführung der Umfangswand 5 aus insbesondere transparentem Material möglich ist.

Im Innenraum 4 des Gehäuses 3 ist ein um die vertikale Maschinenachse MA umlaufend antreibbarer Rotor 10 aufgenommen. Bei der dargestellten Ausführungsform ist der Rotor 10 als kreiszylinderförmige, die Maschinenachse MA konzentrisch umschließende Hohltrommel mit einem Trommelmantel mit käfigartiger Struktur ausgeführt, die von einer Vielzahl von Verschlussaufnahmen 11 zur reihenförmigen Aufnahme jeweils einer Vielzahl von Verschlüssen 2 gebildet ist. Die Verschlussaufnahmen 11, die mit ihrer Längserstreckung jeweils parallel oder im Wesentlichen parallel zur Maschinenachse MA orientiert und an ihrem oberen Ende zum Einbringen der Verschlüsse 2 und an ihrem unteren Ende zum Ausbringen der Verschlüsse 2 offen sind, bilden in ihrer Gesamtheit die käfig- oder gitterartige Struktur bzw. den käfigartigen, in sich stabilen Mantel des Rotors 10 und sind hierfür am Umfang des Rotors 10 in gleichmäßigen Winkel- und Teilsabständen um die Maschinenachse MA verteilt vorgesehen. Im Bereich ihrer oberen und unteren Enden sind die Verschlussaufnahmen 11 jeweils an einem konzentrisch zur Maschinenachse MA angeordneten kreisringförmigen oberen bzw. unteren Tragelement 12, 13 befestigt, und zwar derart, dass die unteren, offenen Enden der Verschlussaufnahmen 11 durch das Tragelement 13 nicht abgedeckt sind, vielmehr im Bereich des unteren Tragelementes 13 oder unterhalb dieses Tragelementes 13 freiliegen.

Die Verschlussaufnahmen 11 können ebenfalls gitterartig ausgeführt sein und jeweils aus mehreren voneinander beabstandeten und parallel zur Maschinenachse MA orientierten stangen- oder stabförmigen Verschlussführungsschienen 14 bestehen, die zwischen sich einen Aufnahmeraum zur reihenförmigen Aufnahme einer Vielzahl von Verschlüssen 2 bilden, und zwar derart, dass die Verschlüsse 2 in jeder Verschlussaufnahme 11 eine sich in einer Achsrichtung parallel zur Maschinenachse MA erstreckende einspurige Verschlussreihe oder -gruppe entlang einer Verschlussachse VA bilden und in der jeweiligen Verschlussaufnahme 11 möglichst weitgehend freiliegen, d.h. nur an einem geringen Teil ihrer Oberfläche von den Verschlussführungsschienen 14 abgedeckt sind. In vergleichbarer Art und Weise können die Verschlussaufnahmen 11 auch als Frästeile mit entsprechend angepassten Konturen ausgeführt werden.

Weiterhin sind die Verschlussaufnahmen 11 bei der dargestellten Ausführungsform so ausgeführt, dass die Verschlüsse 2 in den Verschlussaufnahmen 11 in Bezug auf die Maschinenachse MA eine vorgegebene Orientierung aufweisen, und zwar in der Form, dass sie mit ihrer Verschluss- oder Kappenachse radial zur Maschinenachse MA und beispielsweise mit ihrer offenen Kappenseite radial nach außen orientiert, und zwar in Richtung der Gehäusewand 6, sind.

Mit dem unteren Tragelement 13 ist der Rotor 10 in einem nicht dargestellten Lager an der Unterseite der Sterilisiervorrichtung 1 um die Maschinenachse MA drehbar gelagert und mittels eines Antriebs, beispielsweise einem Elektromotor mit Getriebe um die Maschinenachse MA in Umlaufrichtung A getaktet, schrittweise umlaufend antreibbar.

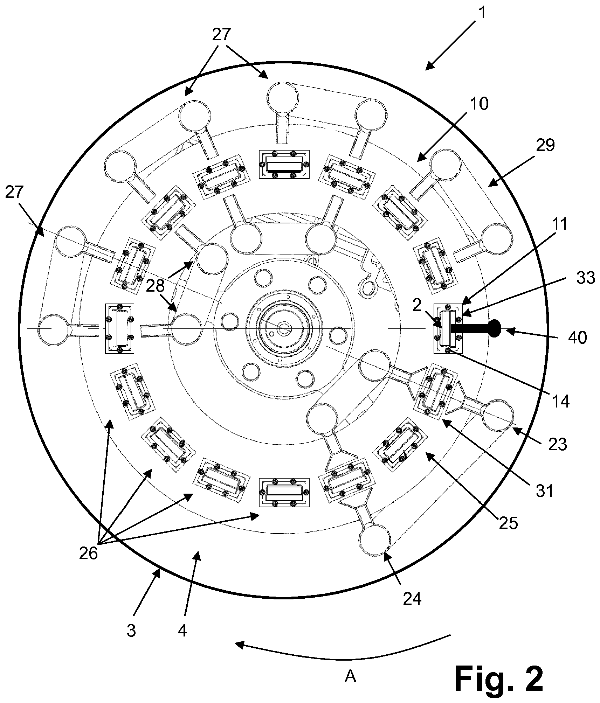

Wie insbesondere aus Figur 2 erkennbar, sind mit dem Rotor 10 nicht umlaufend vorzugsweise in dem Innenraum 4 des Gehäuses 3, d.h. in dem Behandlungsraum der Sterilisiervorrichtung 1, vorzugsweise die mehreren Applikationsstationen 20 vorgesehen, die insbesondere als Sterilisierstation 23, 24 und/oder Trocknungsstation 28 und/oder Aktivierungsstation 27 und/oder Kühlstation 29 ausgebildet sein können, und die vorzugsweise mit ihrer Längserstreckung jeweils parallel zur Maschinenachse MA orientiert sind.

Erfindungsgemäß ist dabei wenigstens einer vertikal ausgerichteten Verschlussaufnahme 11 eine in das Gehäuse 3 zumindest abschnittsweise eingreifende Stellvorrichtung 40 zum Einleiten einer Stellbewegung mittels mindestens eines Stellgliedes 41 auf wenigstens einen in dieser Verschlussaufnahme 11 aufgenommen Verschluss 2 zugeordnet. Nach einem weiteren Merkmal der Erfindung ist dabei das mindestens eine Stellglied 41 über wenigstens einen Faltenbalg 50 mit zumindest einer Öffnung 3.1 des Gehäuses 3 beweglich verbunden.

Dabei kann in der oberen Gehäusewand 6 ein nach schräg oben hin hervortretender Gehäusewandabschnitt 6.1 vorgesehen sein, der mit dem Gehäuse 3 insbesondere einstückig ausgebildet sein kann und dem die zumindest eine Öffnung 3.1 des Gehäuses 3 zugeordnet ist. Der schräg verlaufende Gehäusewandabschnitt 6.1 ist damit in seiner Längserstreckung insbesondere winkelig, d. h. nicht parallel, zur Maschineachse MA orientiert. Unterhalb der Öffnung 3.1, an dem mit dem Gehäuse 3 insbesondere einstückig ausgebildeten Gehäusewandabschnitt 6.1, kann sich dabei der Faltenbalg 50 angeordnet befinden.

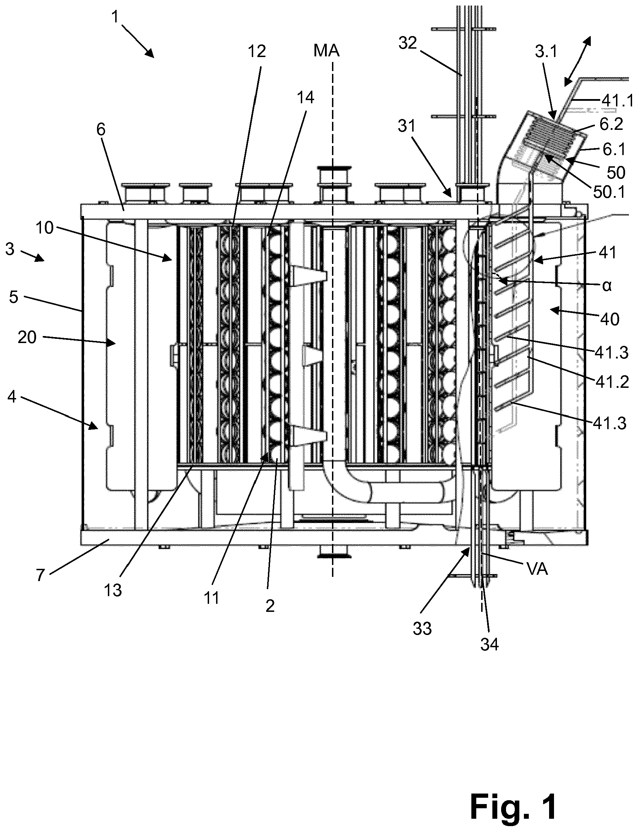

Alternativ kann der vorzugsweise schräg verlaufende Gehäusewandabschnitt 6.1 auch nach oben hin offen ausgebildet und mittels beispielsweise eines Gehäusedeckels 6.2 dicht, insbesondere gas- und/oder flüssigkeitsdicht, verschlossen werden. Mithin kann hierzu der Gehäusedeckel 6.2 die mindestens eine Öffnung 3.1 aufweisen, durch die das Stellglied 41 aus dem Innenraum 4 des Gehäuses 3 nach außen beweglich geführt wird. Die Beweglichkeit des Stellgliedes 41 ist in Figur 1 durch einen Doppelpfeil angedeutet dargestellt. Zur gesteuerten Einleitung der Stellbewegung auf das Stellglied 41 kann beispielsweise ein nicht näher abgebildeter Stellmotor vorgesehen sein.

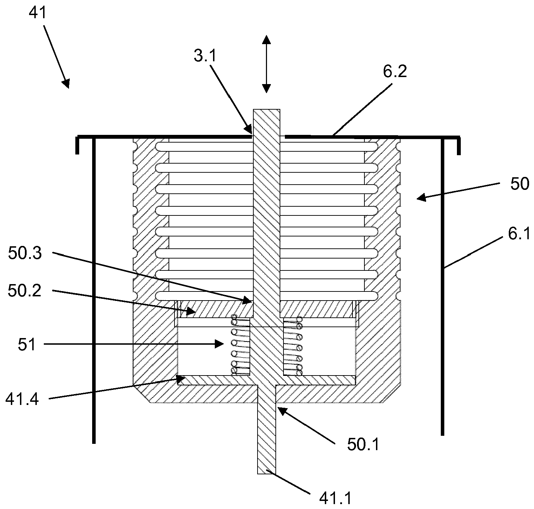

Ferner kann an der Unterseite des Gehäusedeckels 6.2 der Faltenbalg 50 fest als auch dicht mit dem Gehäusedeckel 6.2 verbunden sein. Dies kann beispielweise erfolgen, indem der Gehäusedeckel 6.2 an seiner Innenseite im Bereich der zumindest einen Öffnung 3.1 einen flanschartigen Kragen aufweist, auf den der Faltenbalg 50 aufgeschoben und mit beispielweise einer Muffe befestigt wird. Dadurch ist das Stellglied 41 zwar im Bereich der zumindest einen Öffnung 3.1 von dem Faltenbalg 50 umschlossen, jedoch durch die zumindest eine Öffnung 3.1 beweglich. Zudem sieht der Faltenbalg 50 an einer dem Gehäusedeckel 6.2 abgewandten Seite einen den Faltenbalg 50 durchdringenden Führungsabschnitt 50.1 vor, durch den das Stellglied 41 hindurchführbar ist. Im Bereich bzw. am Führungsabschnitt 50.1 ist das Stellglied 41 in der zu Figur 3 näher beschriebenen Art und Weise fest und dicht an dem Faltenbalg 50 angeordnet, d. h. mit diesem verbunden, und zwar derart, dass sich der Faltenbalg 50 bei Einleitung einer Stellbewegung auf die Stellvorrichtung 40 mit dem Stellglied 41 zumindest in seinem Führungsabschnitt 50.1 mitbewegt. Mittels des Faltenbalges 50 wird damit eine atmosphärische Trennung des Innenraums 4 gegenüber der außerhalb des Gehäuses 3 herrschenden äußeren Atmosphäre erzeugt, und zwar bei gleichzeitiger Beweglichkeit des Stellgliedes 41.

Besonders bevorzugt weist das Stellglied 41 einen ersten, winkelig zur Maschinenachse MA verlaufenden Stellgliedträgerabschnitt 41.1 sowie einen sich an den ersten Stellgliedträgerabschnitt 41.1 anschließenden, im Wesentlichen parallel zur Maschinenachse MA orientierten, zweiten Stellgliedträgerabschnitt 41.2 auf, von dem aus sich vorzugsweise mehrere Stellgliedstoßabschnitte 41.3 kammartig abzweigen. Der erste Stellgliedträgerabschnitt 41.1 wird dabei zumindest teilweise von dem Faltenbalg 50 vorzugsweise konzentrisch umschlossen und befindet sich in diesem aufgenommen. Insbesondere bilden der erste Stellgliedträgerabschnitt 41.1 sowie der zweite Stellgliedträgerabschnitt 41.2 keine Gerade aus, sondern sind winkelig zueinander orientiert angeordnet, und zwar derart, dass sich bei Einleitung einer Stellbewegung auf das Stellglied 41 sowohl eine vertikal, als auch radial ausgebildete Stellbewegung, also eine schräg zur Verschlussachse VA verlaufende Stellbewegung, zumindest der Stellgliedstoßabschnitte 41.3 in Richtung der, der Stellvorrichtung 40 zugeordneten Verschlussaufnahme 11 einstellt.

Bevorzugt kann das Stellglied 41 mit seinen Stellgliedträgerabschnitten 41. 1, 41.2 sowie seinen mehreren Stellgliedstoßabschnitten 41.3 einteilig, bzw. einstückig ausgebildet sein. Alternativ ist es auch möglich, dass die Stellgliedstoßabschnitte 41.3 als separate Bauteile ausgebildet sind, die insbesondere lösbar, beispielweise mittels eines Gewindes verschraubbar, an dem zweiten Stellgliedträgerabschnitt 41.2 angeordnet sind.

Die Anzahl der Stellgliedstoßabschnitte 41.3 sowie deren geometrische Dimensionierung, wie beispielweise der Abstand der einzelnen Stellgliedstoßabschnitte 41.3 untereinander und/oder die Länge und/oder der Durchmesser und/oder der Werkstoff der einzelnen Stellgliedstoßabschnitte 41.3, kann dabei insbesondere an die unterschiedlichen Verschlusstypen sowie weiterhin an die Anzahl der in einer Verschlussaufnahme 11 angeordneten Verschlüsse 2 angepasst sein. Vorzugsweise ist dabei eine in der Anzahl der in einer Verschlussaufnahme 11 maximal aufnehmbaren Anzahl an Verschlüssen 2 entsprechende Anzahl an Stellgliedstoßabschnitten 41.3 vorgesehen. Besonders vorteilhaft ist damit jedem in der Verschlussaufnahme 11 aufnehmbaren Verschlüsse 2 ein separater Stellgliedstoßabschnitt 41.3 zuordenbar.

Darüber hinaus kann es vorteilhaft sein, wenn die einzelnen Stellgliedstoßabschnitte 41.3 mit der Verschlussachse VA einen spitzen Winkel α einschließen, der vorzugsweise zwischen 25° und 85° liegt, besondere bevorzugt näherungsweise 45°beträgt. Weiterhin vorteilhaft spannen die einzelnen Stellgliedstoßabschnitte 41.3 eine gemeinsame Ebene auf, in der sie sich angeordnet befinden.

Wie aus den Figuren 1 und 2 ersichtlich, ist die erfindungsgemäße Stellvorrichtung 40 in ihrer Längserstreckung im Wesentlichen parallel zur Maschinenachse MA orientiert ausgebildet und vorzugsweise in Umlaufrichtung A zwischen der ersten Sterilisierstation 23 und der Kühlstation 29 radial zum Rotor 10 beabstandet angeordnet.

Wie aus Figur 2 ebenfalls erkennbar, können in Umlaufrichtung A des Rotors 10 innen- bzw. außenumfangsseitig mehrere Applikationsstationen 20 vorgesehen sein. Durch die in vorzugsweise festen Winkel- und Teilungsabständen um die Maschinenachse MA angeordneten Verschlussaufnahmen 11 wird der Winkelbetrag festgelegt, um den der Rotor 10 bei der schrittweisen, intermittierenden Drehbewegung um die Maschinenachse MA rotiert. Zwischen den einzelnen Applikationsstationen können auch Verweilstationen 21 vorgesehen sein, an denen keinerlei Behandlung stattfindet, an denen beispielsweise das auf die Verschlüsse 2 in der Applikationsstation 20 aufgebrachte Sterilisiermittel einwirken kann. Bei einer weiteren getakteten Drehung des Rotors 10 können die zuvor an der Verweilstation 21 befindlichen Verschlüsse 2 an eine weitere Applikationsstation 20 gelangen, um dort beispielsweise erneut mit Sterilisiermittel beaufschlagt zu werden.

Beispielsweise kann der ersten Sterilisierstation 23 oberhalb der Bewegungsbahn der Verschlussaufnahme 11 die Verschlussaufgabe 31 zugeordnet sein, die von einem unteren Ende einer äußeren Verschlussförderstrecke 32 gebildet ist und der die zu sterilisierenden Verschlüsse 2 über diese Verschlussförderstrecke 32 durch die obere Gehäusewand 6 zugeführt werden. In der Verschlussförderstrecke 32 weisen die Verschlüsse 2 bereits die ihrer Orientierung in den Verschlussaufnahmen 11 entsprechende Orientierung auf. Ebenso wie die erfindungsgemäße Stellvorrichtung 40 kann in Umlaufrichtung A vor der ersten Sterilisierstation 23 bzw. nach der Kühlstation 29 dabei im Bereich der Unterseite des Gehäuses 3, also an dessen unterer Gehäusewand 7, unterhalb der Bewegungsbahn der Verschlussaufnahmen 11 die Verschlussabgabe 33 vorgesehen sein, die den Einlass für eine nach oben hin offene Verschlussförderstrecke 34 gebildet, welche mit ihrem offenen Ende unterhalb der Bewegungsbahn der Verschlussaufnahmen 11 des Rotors 10 angeordnet ist. Durch die dazu oberhalb zur Verschlussabgabe 33 angeordnete erfindungsgemäße Stellvorrichtung 40 kann damit besonders vorteilhaft eine Stellbewegung auf die in der vertikalen Verschlussaufnahme 11 angeordneten Verschlüsse 2 eingeleitet werden, die über die Verschlussabgabe 33 der Verschlussförderstrecke 34 übergeben werden sollen. Über die Verschlussförderstrecke 34 werden die sterilisierten Verschlüsse 2 der nicht dargestellten Verschließmaschine keimfrei zugeführt.

Bei der dargestellten Ausführungsform sind die Verschlussförderstrecken 32 und 34 jeweils von mehreren, die Verschlüsse 2 zwischen sich aufnehmenden und führenden Führungsschienen gebildet. Weiterhin weisen bei der dargestellten Ausführungsform die Verschlussförderstrecken 32 und 34 zumindest in der Nähe des Rotors 10 jeweils einen vertikalen Verlauf auf, sodass die Verschlüsse 2 in den Verschlussförderstrecken 32 und 34 allein schon aufgrund ihrer Schwerkraft bewegt bzw. gefördert werden. Um allerdings ein erneutes Verkeimen der sterilisierten Verschlüsse 2 auf der Verschlussförderstrecke 34 zu vermeiden ist diese in einer nicht dargestellten Ummantelung oder Einhausung aufgenommen, die bevorzugt mit einem Überdruck eines sterilen gas- und/oder dampfförmigen Mediums, beispielsweise mit dem Überdruck steriler Luft beaufschlagt ist.

Die Verschlussaufgabe 31 und die Verschlussabgabe 33 sind bezogen auf die Maschinenachse MA in einem Winkelabstand, der einem Vielfachen des Teilungsabstandes der Verschlussaufnahmen 11 am Rotor 10 entspricht, so angeordnet, dass immer dann, wenn sich eine Verschlussaufnahme 11 mit ihrem oberen offenen Ende in der Stillstandsphase der Drehbewegung des Rotors 10 an der Verschlussaufgabe 31 befindet, sich eine andere Verschlussaufnahme 11 mit ihrem unteren offenen Ende an der Verschlussabgabe 33 befindet. Weiterhin ist die Verschlussabgabe 33 bezogen auf die Umlaufrichtung A des Rotors 10 um einen möglichst großen Winkelbetrag, beispielsweise um einen Winkelbetrag etwas kleiner als 360°, z.B. um einen Winkelbetrag von 330° oder von etwa 330° von der Verschlussaufgabe 31 beabstandet, um eine möglichst lange Behandlungsstrecke und damit auch bei hohen Leistungen der Vorrichtung 1, d.h. bei einer hohen Anzahl der je Zeiteinheit mit dieser Vorrichtung 1 sterilisierten Verschlüssen 2 bzw. bei einer entsprechend hohen Drehgeschwindigkeit des Rotors 10 eine möglichst lange Behandlungsdauer für das Entkeimen bzw. Sterilisieren der Verschlüsse 2 zu erreichen, und zwar bei reduzierten Durchmesser des Rotors 10 und kompakter Bauform der Vorrichtung 1 insgesamt. Vorzugsweise ist die Verschlussaufgabe 31 oberhalb der ersten Sterilisierstation 23 angeordnet, wohingegen die Verschlussabgabe 33 einen Winkel- bzw. Teilungsabstand vor der Sterilisierstation 23 angeordnet ist.

Mit einem nicht näher dargestellten Antrieb wird der Rotor 10 getaktet um die Maschinenachse MA angetrieben und zwar derart, dass der Rotor 10 in jeder Bewegungsphase der getakteten Drehbewegung einen dem Teilungsabstand zweier Verschlussaufnahmen 11 entsprechenden Drehschritt ausführt und sich in jeder Stillstandsphase der getakteten Drehbewegung eine leere Verschlussaufnahme 11 unterhalb der Verschlussaufgabe 31 befindet, über die dann aus der Verschlussförderstrecke 32 die Verschlussaufnahme 11 vollständig mit Verschlüssen 2 gefüllt wird. Mit der getakteten Drehbewegung des Rotors 10 werden die jeweils mit den Verschlüssen 2 gefüllten Verschlussaufnahmen 11 entlang der Behandlungsstationen der Behandlungsstrecke bewegt und dabei in den Stillstandsphasen des Rotors 10 an den einzelnen Stationen behandelt und dabei entkeimt.

Immer dann, wenn eine Verschlussaufnahme 11 des Rotors 10 die Verschlussabgabe 33 erreicht hat, fallen sämtliche, sterilisierten Verschlüsse 2 dieser Verschlussaufnahme über die Verschlussabgabe 33 in die Verschlussförderstrecke 34, über die die entkeimten bzw. sterilisierten Verschlüsse 2 dann der weiteren Verwendung zugeführt werden.

Figur 3 zeigt einen Ausschnitt der erfindungsgemäßen Vorrichtung 1 gemäß Figur 1 des Gehäusewandabschnittes 6.1 mit dem zumindest einen Faltenbalg 50 in einer seitlichen Schnittdarstellung. Dabei kann die zumindest eine Öffnung 3.1 des Gehäuses 3 insbesondere dem Gehäusedeckel 6.2 zugeordnet sein, durch den das Stellglied 41 mit seinem ersten Stellgliedträgerabschnitt 41.1 aus dem Innenraum 4 des Gehäuses 3 nach außen beweglich geführt ist. An der Unterseite des Gehäusedeckels 6.2 kann sich der zumindest eine Faltenbalg 50 fest und dicht, insbesondere gas- und/oder flüssigkeitsdicht, angeordnet befinden.

Der erste Stellgliedträgerabschnitt 41.1 kann dabei insbesondere an einer ersten Seite durch die zumindest eine Öffnung 3.1, relativ zu dieser beweglich, hindurchgeführt sein, während er mit seiner zweiten Seite zwar durch den Führungsabschnitt 50.1 hindurch, jedoch in bzw. an diesem fest, also mechanisch fixiert, aufgenommen ist und zwar derart, dass sich der Faltenbalg 50 bei Einleitung einer Stellbewegung auf den ersten Stellgliedträgerabschnitt 41.1 zumindest mit seinem Führungsabschnitt 50.1 mitbewegt. Mittels des Faltenbalges 50 wird damit eine atmosphärische Trennung des Innenraums 4 gegenüber der außerhalb des Gehäuses 3 herrschenden, äußeren Atmosphäre erzeugt, und zwar bei gleichzeitiger Beweglichkeit des Stellgliedes 41.

Für eine verbesserte Lagebestimmung des Stellgliedes 41, kann sich im Inneren des Faltenbalges 50 ein Führungsmittel 50.2, das beispielweise als ringartiger Kolben ausgebildet sein kann, angeordnet befinden. Insbesondere ist das Führungsmittel 50.2 fest, also lagefixiert, im Inneren des Faltenbalges 50 aufgenommen und weist eine Durchbrechung 50.3 auf, die in ihrer Dimensionierung an das Stellglied 41, insbesondere an den ersten Stellgliedträgerabschnitt 41.1 derart angepasst ist, dass das Stellglied 41 durch die Durchbrechung 50.3 hindurchgeführt werden kann.

Ferner kann das Stellglied 41, insbesondere dessen erster Stellgliedträgerabschnitt 41.1, einen näherungsweise rechtwinklig radial vom Stellglied 41 nach außen hin abstehenden Flügelabschnitt 41.4 aufweisen, der an dem Faltenbalg 50 fest, insbesondere auch dicht, fixiert ist. Zwischen dem Flügelabschnitt 41.4 und dem Führungsmittel 50.2 kann eine Feder 51, die insbesondere als spiralförmige Druckfeder ausgebildet sein kann, unter einer definierten Vorspannung um das Stellglied 41 herum aufgenommen werden.

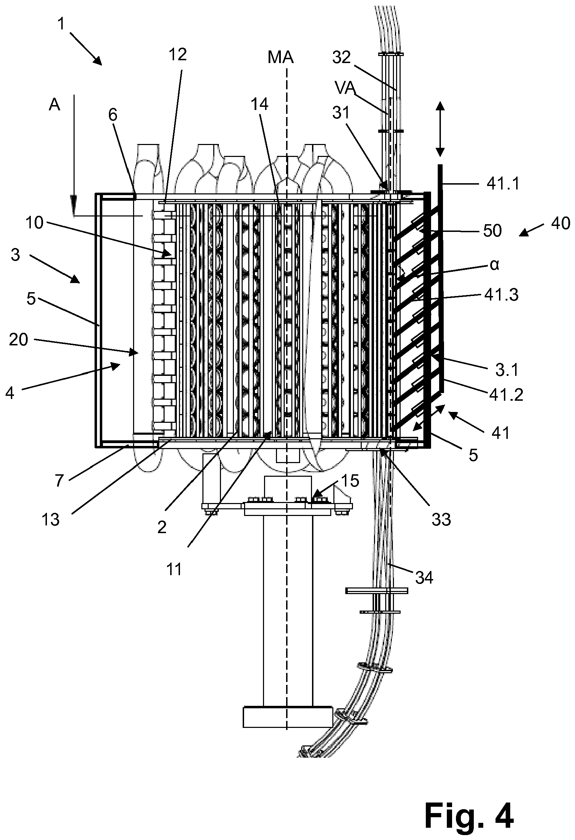

Figur 4 zeigt in seitlicher schematischer Darstellung eine weitere Ausführungsvariante der erfindungsgemäßen Vorrichtung 1 zum Sterilisieren von Verschlüssen, bei der das Stellglied 41 mehrere Stellgliedstoßabschnitte 41.3 aufweist, denen jeweils ein separater Faltenbalg 50 zugeordnet ist. Die Stellgliedstoßabschnitte 41.3 sind dabei jeweils durch eine separate Öffnung 3.1 des Gehäuses 3, insbesondere der Umfangswand 5, beweglich hindurchgeführt und mittels des jeweiligen Faltenbalges 50 beweglich mit der entsprechenden Öffnung 3.1 verbunden. Das Stellglied 41 wird mit seinen mehreren Stellgliedstoßanschnitten 41.3 insbesondere von außen durch die mehreren Öffnungen 3.1 in den Innenraum 4 der Sterilisiervorrichtung 1 beweglich hindurchgeführt. Einem jedem Stellgliedstoßanschnitt 41.3 kann dabei im Innenraum 4 ein separater Faltenbalg 50 zugeordnet sein, der den jeweiligen Stellgliedstoßabschnitt 41.3 umschließt, bzw. zumindest teilweise aufnimmt. Ferner kann der jeweilige Faltenbalg 50 mit seiner ersten Seite fest als auch dicht an der Innenseite der Umfangswand 5 angeordnet, insbesondere mit dieser verbunden sein. Die jeweiligen Stellgliedstoßabschnitte 41.3 können durch die entsprechenden Öffnungen 3.1 beweglich hindurchführt werden. Damit sind die jeweiligen Stellgliedstoßabschnitte 41.3 auch relativ beweglich zur ersten Seite der entsprechenden Faltenbalge 50 ausgebildet. Weiterhin werden die mehreren Stellgliedstoßabschnitte 41.3 mechanisch fixiert in den jeweiligen Führungsabschnitten 50.1 der entsprechenden Faltenbalge 50 aufgenommen, so dass sich bei Einleitung einer Stellbewegung der jeweilige Führungsabschnitt 50.1 mit dem entsprechenden Stellgliedstoßabschnitt 41.3 mitbewegt. Dabei kann vorgesehen sein, dass der jeweilige Faltenbalg 50 den entsprechenden Stellgliedstoßabschnitt 41.3 im Innenraum 4 des Gehäuses 3 vollständig umschließt. Hierbei weist in dieser Ausführungsvariante der Faltenbalg 50 insbesondere auch keinen ihn durchdringenden Aufnahmeabschnitt 50.1 auf. Alternativ kann der jeweilige Stellgliedstoßabschnitt 41.3 auch durch den entsprechenden Führungsabschnitt 50.1 des jeweiligen Faltenbalges 50 hindurchgeführt und in diesem fest gehalten sein.

Die Anzahl der Stellgliedstoßabschnitte 41.3 sowie deren geometrische Dimensionierung, wie beispielweise der Abstand der einzelnen Stellgliedstoßabschnitte 41.3 untereinander und/oder die Länge und/oder der Durchmesser und/oder deren Werkstoff, kann dabei insbesondere an die unterschiedlichen Verschlusstypen sowie weiterhin an die Anzahl der in einer Verschlussaufnahme 11 angeordneten Verschlüsse 2 angepasst sein. Vorzugsweise ist dabei eine in der Anzahl der in einer Verschlussaufnahme 11 maximal aufnehmbaren Anzahl an Verschlüssen 2 entsprechende Anzahl an Stellgliedstoßabschnitten 41.3 vorgesehen. Besonders vorteilhaft ist damit jedem in der Verschlussaufnahme 11 aufnehmbaren Verschluss 2 ein separater Stellgliedstoßabschnitt 41.3 zuordenbar.

Dabei sind die dieser Ausführungsvariante der Figur 4 der erste und zweite Stellgliedträgerabschnitt 41.1, 41.2 des Stellgliedes 41 stabförmig, d. h. gerade, ausgebildet, also unterschiedlich zu der Ausführungsvariante der Figur 1 insbesondere nicht winkelig. Zur Einleitung einer gesteuerten Stellbewegung auf das Stellglied 41 kann dieses außerhalb des Gehäuses 3 mit einem nicht näher abgebildeten Stellmotor zusammenwirken, derart, dass sich durch Einleitung einer Stellbewegung auf das Stellglied 41 sowohl eine vertikal, als auch radial ausgebildete Stellbewegung, also eine schräg zur Verschlussachse VA verlaufende Stellbewegung zumindest der Stellgliedstoßabschnitte 41.3 in Richtung der, der Stellvorrichtung 40 zugeordneten Verschlussaufnahme 11 einstellt. Die Stellbeweglichkeit des Stellglieds 41 ist in Figur 4 schematisch mit einem Doppelpfeil angedeutet dargestellt.

Auch bei der Ausführungsvariante der Figur 4 ist es vorteilhaft, wenn die einzelnen Stellgliedstoßabschnitte 41.3 eines jeden Stellglieds 41 mit der Verschlussachse VA einen spitzen Winkel α einschließen, der vorzugsweise zwischen 25° und 85° liegt, besondere bevorzugt näherungsweise 45°beträgt. Weiterhin vorteilhaft spannen die einzelnen Stellgliedstoßabschnitte 41.3 eine gemeinsame Ebene auf, in der sie sich angeordnet befinden.

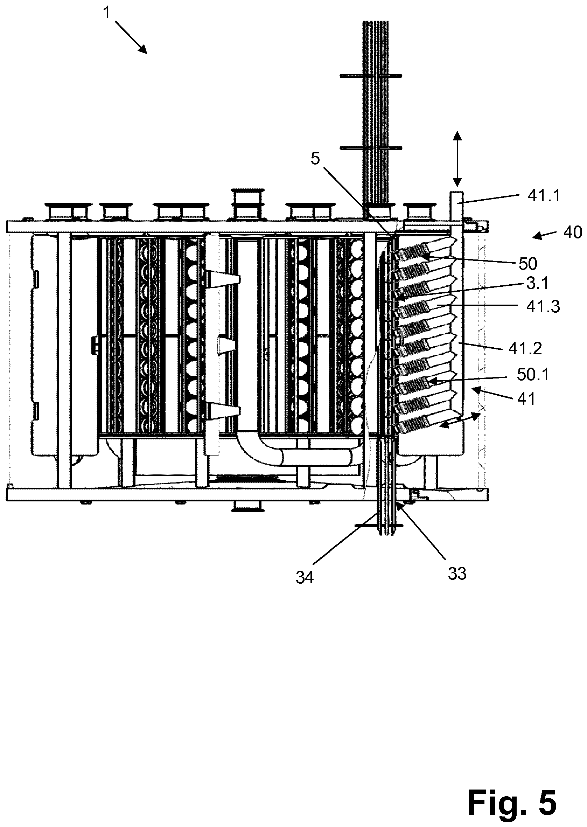

Figur 5 zeigt in seitlicher schematischer Darstellung eine nochmals weitere Ausführungsvariante der erfindungsgemäßen Vorrichtung 1 zum Sterilisieren von Verschlüssen, bei der unterschiedlich zu der Ausführungsvariante der Figur 4 die mehreren Faltenbalge 50 nicht im Innenraum 4 des Gehäuses 3, sondern außerhalb, insbesondere an der Außenseite der nur stilisiert dargestellten Umfangswand 5, vorgesehen sind. Die jeweiligen Faltenbalge 50 sind mit ihrer ersten Seite an den entsprechenden Öffnungen 3.1 der Umfangswand 5 fest angeordnet. Beispielweise können die Faltenbalge 50 hierfür mit ihrer ersten Seite mittels einer nicht näher dargestellten Muffe an einem an der entsprechenden Öffnung 3.1 vorgesehen flanschartigen Kragen befestigt sein. Die jeweiligen Stellgliedstoßabschnitte 41.3 können durch die entsprechenden Öffnungen 3.1 beweglich hindurchführt werden. Damit sind die jeweiligen Stellgliedstoßabschnitte 41.3 auch relativ beweglich zur ersten Seite der entsprechenden Faltenbalge 50 ausgebildet. Weiterhin werden die mehreren Stellgliedstoßabschnitte 41.3 mechanisch fixiert in den jeweiligen Führungsabschnitten 50.1 der entsprechenden Faltenbalge 50 aufgenommen, so dass sich bei Einleitung einer Stellbewegung der jeweilige Führungsabschnitt 50.1 mit dem entsprechenden Stellgliedstoßabschnitt 41.3 mitbewegt mit bewegt.

Auch die Stellvorrichtung 40 der Ausführungsvariante der Figur 5 kann zur Einleitung einer gesteuerten Stellbewegung auf das Stellglied 41 mit einem nicht näher abgebildeten Stellmotor zusammenwirken, derart, dass sich durch Einleitung einer Stellbewegung auf das Stellglied 41 sowohl eine vertikal, als auch radial ausgebildete Stellbewegung, also eine schräg zur Verschlussachse VA verlaufende Stellbewegung zumindest der Stellgliedstoßabschnitte 41.3 in Richtung der, der Stellvorrichtung 40 zugeordneten Verschlussaufnahme 11 einstellt. Die Stellbeweglichkeit des Stellglieds 41 ist in Figur 5 schematisch mit einem Doppelpfeil angedeutet dargestellt.

### Bezugszeichenliste

- 1: Sterilisiervorrichtung
- 2: Verschluss
- 3: Gehäuse
- 3.1: Öffnung
- 4: Innenraum
- 5: Umfangswand
- 6: Obere Gehäusewand
- 6.1: Gehäusewandabschnitt
- 6.2: Gehäusedeckel
- 7: Untere Gehäusewand

- 10: Rotor
- 11: Verschlussaufnahme
- 12: oberes Tragelement
- 13: unteres Tragelement
- 14: Verschlussführungsschiene
- 16: Verschlussanlagefläche

- 20: Applikationsstation
- 23: Sterilisierstation
- 24: Sterilisierstation
- 25: Verweilstation
- 26: Verweilstation
- 27: Aktivierungsstation
- 28: Trocknungsvorrichtung
- 29: Kühlstation
- 31: Verschlussaufgabe
- 32: Verschlussförderstrecke
- 33: Verschlussabgabe
- 34: Verschlussförderstrecke
- 40: Stellvorrichtung
- 41: Stellglied
- 41.1: erster Stellgliedträgerabschnitt
- 41.2: zweiter Stellgliedträgerabschnitt
- 41.3: Stellgliedstoßabschnitt
- 41.4: Flügelabschnitt

- 50: Faltenbalg
- 50.1: Führungsabschnitt
- 50.2: Führungsmittel
- 50.3: Durchbrechung
- 51: Feder
- 52: Verbindungsträger

- A: Umlaufrichtung des Rotors 10
- MA: Maschinenachse
- VA: Verschlussachse

## Patentansprüche

1. Vorrichtung zum Sterilisieren und/oder Entkeimen von Verschlüssen (2) zum Verschließen von Flaschen oder Behältern, mit einem Transportsystem zum Bewegen der Verschlüsse durch (2) wenigstens eine Behandlungszone, in der die Verschlüsse (2) sterilisiert und/oder getrocknet werden, wobei das Transportsystem in der wenigstens einen Behandlungszone wenigstens einen um eine Maschinen- oder Rotorachse (MA) umlaufend antreibbaren Rotor (10) mit einer Vielzahl von am Umfang dieses Rotors (10) ausgebildeten, vertikal ausgerichteten Verschlussaufnahmen (11) aufweist, mittels denen die Verschlüsse (2) durch die wenigstens eine Behandlungszone auf einer Behandlungsstrecke zwischen einer Verschlussaufgabe (31) und einer Verschlussabgabe (33) bewegt werden, und wobei die Vorrichtung innerhalb eines Gehäuses (3) aufgenommen ist,
**dadurch gekennzeichnet,**
**dass** wenigstens einer vertikal ausgerichteten Verschlussaufnahme (11) eine in das Gehäuse (3) zumindest abschnittsweise eingreifende Stellvorrichtung (40) zum Einleiten einer Stellbewegung mittels mindestens eines Stellglieds (41) auf wenigstens einen in dieser Verschlussaufnahme (11) aufgenommenen Verschluss (2) zugeordnet ist, und wobei das mindestens eine Stellglied (41) über wenigstens einen Faltenbalg (50) mit zumindest einer Öffnung (3.1) des Gehäuses (3) beweglich verbunden ist.

2. Vorrichtung nach Anspruch 1, **dadurch gekennzeichnet, dass** die Stellvorrichtung (40) in ihrer Längserstreckung parallel zur Maschinenachse (MA) orientiert ausgebildet und radial zum Rotor (10) beabstandet angeordnet ist.

3. Vorrichtung nach Anspruch 1 oder 2, **dadurch gekennzeichnet, dass** die Stellvorrichtung (40) oberhalb der Verschlussabgabe (33) derart angeordnet ist, dass mittels des mindestens eines Stellgliedes (41) wenigstens ein in der Verschlussaufnahme (11) aufgenommener Verschluss (2) mit einer Stellbewegung beaufschlagbar ist.

4. Vorrichtung nach Anspruch 1 bis 3, **dadurch gekennzeichnet, dass** das Stellglied (41) zur Einleitung einer Stellbewegung wenigstens einen Stellgliedstoßabschnitt (41.3) aufweist, der mit einer Verschlussachse (VA) einen spitzen Winkel (α) einschließt, der vorzugsweise zwischen 25°und 85° Grad liegt.

5. Vorrichtung nach Anspruch 1 bis 4, **dadurch gekennzeichnet, dass** das Stellglied (41) zumindest einen ersten und zweiten Stellgliedträgerabschnitt (41.2) aufweist, wobei der zweite Stellgliedträgerabschnitt (41.2) mit dem zumindest einen Stellgliedstoßabschnitt (41.3) verbunden ist

6. Vorrichtung nach Anspruch 5, **dadurch gekennzeichnet, dass** an dem zweiten Stellgliedträgerabschnitt (41.2) mehrere Stellgliedstoßabschnitte (41.3) angeordnet sind.

7. Vorrichtung nach Anspruch 5 oder 6, **dadurch gekennzeichnet, dass** die Stellgliedträgerabschnitte (41.2, 41.3) stabförmig ausgebildet sind.

8. Vorrichtung nach Anspruch 5 oder 6, **dadurch gekennzeichnet, dass** der zweite Stellgliedträgerabschnitt (41.2) sich an den ersten Stellgliedträgerabschnitt (41.1) anschließt, wobei der erste Stellgliedträgerabschnitt (41.1) und der zweite Stellgliedträgerabschnitt (41.2) winkelig zueinander angeordnet sind.

9. Vorrichtung nach Anspruch 5 bis 8, **dadurch gekennzeichnet, dass** das Stellglied (41) mit seinem ersten Stellgliedträgerabschnitt (41.1) zumindest teilweise in dem Faltenbalg (50) aufgenommen und durch die Öffnung (3.1) des Gehäuses (3) beweglich geführt ist, vorzugsweise gas- und/oder flüssigkeitsdicht.

10. Vorrichtung nach einem der Ansprüche 5 bis 9, **dadurch gekennzeichnet, dass** der erste Stellgliedträgerabschnitt (41.1) an einer ersten Seite durch die zumindest eine Öffnung (3.1) des Gehäuses (3) beweglich zu dieser hindurchgeführt ist, und mit seiner zweiten Seite durch einen Führungsabschnitt (50.1) des wenigstens einen Faltenbalges (50) hindurchgeführt und in dem Führungsabschnitt (50.1) mechanisch fixiert aufgenommen ist.

11. Vorrichtung nach Anspruch 4 bis 8, dass das Stellglied (41) mit seinem wenigstens einen Stellgliedstoßabschnitt (41.3) zumindest teilweise in wenigstens einem Faltenbalg (50) aufgenommen ist und durch die wenigstens eine Öffnung (3.1) des Gehäuses (3) beweglich geführt ist, vorzugsweise gas- und/oder flüssigkeitsdicht.

12. Vorrichtung nach Anspruch 11, dass jeweils einem Stellgliedstoßabschnitt (41.3) ein separater Faltenbalg (50) zugeordnet ist, wobei die einzelnen Stellgliedstoßabschnitte (41.3) über jeweils eine separate Öffnung (3.1) des Gehäuses (3) geführt und über den jeweiligen Faltenbalg (50) beweglich verbunden sind.

## Claims

1. Device for sterilising and/or bactericidal treatment of closures (2) for closing bottles or containers, with a transport system for moving the closures (2) through at least one treatment zone, in which the closures (2) are sterilised and/or dried, wherein the transport system, in the at least one treatment zone, comprises at least one rotor (10) which can be driven such as to circulate about a machine axis or rotor axis (MA), with a plurality of closure receivers (11) formed at the circumference of this rotor (10) and oriented vertically, by means of which the closures (2) are moved through the at least one treatment zone on a treatment segment between a closure inlet (31) and a closure outlet (33), and wherein the device is accommodated inside a housing (3),
**characterised in that**
at least one vertically oriented closure receiver (11) is assigned an actuating device (40) which engages at least partially in the housing (3) for the purpose of applying an actuation movement, by means of at least one actuator (41), to a closure (2) received in this closure receiver (11), and wherein the at least one actuator (41) is movably connected by means of at least one bellows (50) to at least one opening (3.1) of the housing (3).

2. Device according to claim 1, **characterised in that** the actuating device (40) is configured as oriented in its longitudinal extension parallel to the machine axis (MA) and is arranged radially at a distance from the rotor (10).

3. Device according to claim 1 or 2, **characterised in that** the actuating device (40) is arranged above the closure outlet (33) in such a way that, by means of the at least one actuator (41), at least one closure (2) received in the closure receiver (11) can be subjected to an actuation movement.

4. Device according to any one of claims 1 to 3, **characterised in that**, in order to initiate an actuation movement, the actuator (41) comprises at least one actuator impact section (41.3), which encloses with a closure axis (VA) an acute angle α, which preferably lies between 25° and 85°.

5. Device according to any one of claims 1 to 4, **characterised in that** the actuator (41) comprises at least one first and second actuator carrier section (41.2), wherein the second actuator carrier section (41.2) is connected to the at least one actuator impact section (41.3).

6. Device according to claim 5, **characterised in that** a plurality of actuator impact sections (41.3) are arranged at the second actuator carrier section (41.2).

7. Device according to claim 5 or 6, **characterised in that** the actuator carrier sections (41.2, 41.3) are configured in rod or bar shape.

8. Device according to claim 5 or 6, **characterised in that** the second actuator carrier section (41.2) connects to the first carrier section (41.1), wherein the first actuator carrier section (41.1) and the second actuator carrier section (41.2) are arranged at an angle to one another.

9. Device according to any one of claims 5 to 8, **characterised in that** the actuator (41) is accommodated with its first actuator carrier section (41.1) at least partially in the bellows (50) and is movably guided through the opening (3.1) of the housing (3), preferably gas-tight and/or fluid-tight.

10. Device according to any one of claims 5 to 9, **characterised in that** the first actuator carrier section (41.1), at a first side, is movably guided through the at least one opening (3.1) of the housing (3), and, with its second side, is guided through a guide section (50.1) of the at least one bellows (50) and accommodated, mechanically fixed, in the guide section (50.1).

11. Device according to any one of claims 4 to 8, **characterised in that** the actuator (41) is accommodated with its at least one actuator impact section (41.3) at least partially in at least one bellows (50), and is movably guided through the at least one opening (3.1) of the housing (3), preferably gas-tight and/or fluid-tight.

12. Device according to claim 11, **characterised in that** in each case an actuator impact section (41.3) is assigned a separate bellows (50), wherein the individual actuator impact sections (41.3) are guided in each case via a separate opening (3.1) of the housing (3), and are movably connected by way of the respective bellows (50).

## Revendications

1. Dispositif servant à stériliser et/ou à désinfecter des systèmes de fermeture (2) servant à fermer des bouteilles ou des contenants, avec un système de transport servant à déplacer les systèmes de fermeture (2) à travers au moins une zone de traitement, dans laquelle les systèmes de fermeture (2) sont stérilisés et/ou séchés, dans lequel le système de transport présente, dans l'au moins une zone de traitement, au moins un rotor (10) pouvant être entraîné en rotation autour d'un axe de machine ou de rotor (MA) avec une pluralité de logements de système de fermeture (11) réalisés au niveau de la périphérie dudit rotor (10), orientés de manière verticale, au moyen desquels les systèmes de fermeture (2) sont déplacés à travers l'au moins une zone de traitement sur une ligne de traitement entre une entrée de système de fermeture (31) et une sortie de système de fermeture (33), et dans lequel le dispositif est logé à l'intérieur d'un boîtier (3),
**caractérisé en ce**
**qu'**un dispositif de réglage (40) venant en prise au moins par endroits avec le boîtier (3) servant à imprimer un déplacement de réglage au moyen d'au moins un organe de réglage (41) sur au moins un système de fermeture (2) logé dans ledit logement de fermeture (11) est associé à au moins un logement de système de fermeture (11) orienté de manière verticale, et dans lequel l'au moins un organe de réglage (41) est relié de manière mobile à au moins une ouverture (3.1) du boîtier (3) par l'intermédiaire d'au moins un soufflet à plis (50).

2. Dispositif selon la revendication 1, **caractérisé en ce que** le dispositif de réglage (40) est réalisé de manière orientée dans son extension longitudinale de manière parallèle par rapport à l'axe de machine (MA) et disposé de manière espacée radialement par rapport au rotor (10).

3. Dispositif selon la revendication 1 ou 2, **caractérisé en ce que** le dispositif de réglage (40) est disposé au-dessus de la sortie de système de fermeture (33) de telle manière qu'au moins un système de fermeture (2) logé dans le logement de système de fermeture (11) peut être soumis à l'action d'un déplacement de réglage au moyen de l'au moins un organe de réglage (41).

4. Dispositif selon la revendication 1 à 3, **caractérisé en ce que** l'organe de réglage (41) servant à imprimer un déplacement de réglage présente au moins une section de jonction d'organe de réglage (41.3), qui forme avec un axe de système de fermeture (VA) un angle aigu (a), qui est compris de préférence entre 25° et 85° degrés.

5. Dispositif selon la revendication 1 à 4, **caractérisé en ce que** l'organe de réglage (41) présente au moins une première et une deuxième section de support d'organe de réglage (41.2), dans lequel la deuxième section de support d'organe de réglage (41.2) est reliée à l'au moins une section de jonction d'organe de réglage (41.3).

6. Dispositif selon la revendication 5, **caractérisé en ce que** plusieurs sections de jonction d'organe de réglage (41.3) sont disposées au niveau de la deuxième section de support d'organe de réglage (41.2).

7. Dispositif selon la revendication 5 ou 6, **caractérisé en ce que** les sections de support d'organe de réglage (41.2, 41.3) sont réalisées de manière à présenter une forme de barre.

8. Dispositif selon la revendication 5 ou 6, **caractérisé en ce que** la deuxième section de support d'organe de réglage (41.2) se raccorde à la première section de support d'organe de réglage (41.1), dans lequel la première section de support d'organe de réglage (41.1) et la deuxième section de support d'organe de réglage (41.2) sont disposées de manière angulaire l'une par rapport à l'autre.

9. Dispositif selon la revendication 5 à 8, **caractérisé en ce que** l'organe de réglage (41) est logé par sa première section de support d'organe de réglage (41.1) au moins en partie dans le soufflet à plis (50) et guidé de manière mobile à travers l'ouverture (3.1) du boîtier (3), de préférence de manière étanche aux gaz et/ou aux liquides.

10. Dispositif selon l'une quelconque des revendications 5 à 9, **caractérisé en ce que** la première section de support d'organe de réglage (41.1) est guidée de manière mobile au niveau d'un premier côté à travers l'au moins une ouverture (3.1) du boîtier (3) de part en part et est guidée par son deuxième côté à travers une section de guidage (50.1) de l'au moins un soufflet à plis (50) de part en part et logée avec un blocage mécanique dans la section de guidage (50.1).

11. Dispositif selon la revendication 4 à 8, **caractérisé en ce que** l'organe de réglage (41) est logé par son au moins une section de jonction d'organe de réglage (41.3) au moins en partie dans au moins un soufflet à plis (50) et est guidé de manière mobile à travers l'au moins une ouverture (3.1) du boîtier (3) de part en part, de préférence de manière étanche aux gaz et/ou aux liquides.

12. Dispositif selon la revendication 11, **caractérisé en ce qu'**un soufflet à plis (50) séparé est associé à respectivement une section de jonction d'organe de réglage (41.3), dans lequel les diverses sections de jonction d'organe de réglage (41.3) sont guidées par respectivement une ouverture (3.1) séparée du boîtier (3) et reliées de manière mobile par l'intermédiaire du soufflet à plis (50) séparé.
